# EUROPEAN PATENT APPLICATION

(11) **EP 4 763 215 A2**
(43) Date of publication of application: **24.06.2026**
(21) Application number: 26176691.9
(22) Date of filing: 08.10.2020
(51) Int. Cl.: A61K 38/00

(54) **ENHANCEMENT AND STABILISATION OF PROTEOLYTIC ACTIVITY OF PROTEASES**

(30) Priority: 09.10.2019 AU 2019903801
(62) Divisional of application: 20874513.3
(71) Applicant: Phoenix Eagle Company Pty Ltd, Subiaco, WA 6008 (AU)
(72) Inventor: TRIGIANTE, Giuseppe, Watford WD17 1QQ (GB)
(74) Representative: Haseltine Lake Kempner LLP

(57) **Abstract**

The present invention relates to methods for the enhancement and stabilisation of the proteolytic activity of proteases, methods for producing compositions comprising proteases with proteolytic activity that has been enhanced and stabilised, compositions comprising proteases with proteolytic activity that has been enhanced and stabilised obtained or obtainable by the aforementioned methods, use of such compositions in the manufacture of medicaments and cosmetics, the use of such compositions in the treatment of diseases and disorders including wounds, and in cosmetic applications, and associated kits.

## Description

### Field of the Invention

The present invention relates to methods for the enhancement and stabilisation of the proteolytic activity of proteases, methods for producing compositions comprising proteases with proteolytic activity that has been enhanced and stabilised, compositions comprising proteases with proteolytic activity that has been enhanced and stabilised obtained or obtainable by the aforementioned methods, use of such compositions in the manufacture of medicaments and cosmetics, use of such compositions in the treatment of diseases and disorders including wounds, and in cosmetic applications, and associated kits.

### Background to the Invention

Papain and bromelain are proteases used in pharmaceutical products for debridement and in cosmetic/cosmeceutical products for exfoliation and skin lightening. It is the proteolytic activity of papain and bromelain that brings about such debridement, exfoliation and skin lightening. However, neither papain nor bromelain preparations have fulfilled their potential due to the instability of their proteolytic activity.

Considerable effort has previously been invested in developing stable compositions that promote wound healing through the removal of dead and damaged tissue such as that found in wounds, such as burns and chronic ulcers. Efficient debridement is essential since dead and dying tissue is an excellent culture medium for opportunistic infections. Septicemia resulting from infections is the major cause of death in severely burned patients.

One previous approach has been to use proteolytic enzymes such as papain, trypsin and bromelain. In particular, NexoBrid^{™}, which is a concentrate of proteolytic enzymes enriched in bromelain, was approved in Europe in 2012 for the removal of eschar (i.e. debridement) in adults with deep partial- and full-thickness thermal burns. However, the European Medicines Agency confirmed in its "*Assessment report - Nexobrid - Concentrate of proteolytic enzymes enriched in bromelain*" 20 September 2012 under section 2.2.3, page 14, that a common problem with compositions of proteolytic enzymes is the poor stability of proteolytic activity, stating that:
"New compatibility (in-use stability) studies at 25°C and 37°C have been performed demonstrating that NexoBrid degrades within hours after mixing. The applicant's conclusion that the product should be used immediately after mixing is therefore supported."

Accordingly, NexoBrid^{™} is typically supplied as a lyophilised powder which is reconstituted prior to use with a gel vehicle and must be used within 15 minutes of formulation. It would therefore be advantageous to provide compositions such as NexoBrid^{™} in a ready-for-use form and without the need for reconstitution. However, to do so would require a significant enhancement of the stability of the debriding activity in order to achieve an acceptable shelf life.

Similarly, in cosmetic products containing papain and/or bromelain that are marketed as having exfoliating and/or skin lightening properties, the exfoliating activity is often severely reduced in a short period of time due to the loss of activity of the papain and/or bromelain in the cosmetic products.

Methods have previously been devised to address the problem of loss of proteolytic activity of proteases in pharmaceutical products, including storing the enzymes at a pH where the loss of activity is low or absent (in the case of papain, an acidic pH has been used) or in a solid form. These treatments, however, require expert end-user processing to obtain a viable product. This limits the usefulness of such methods and compositions.

Another avenue previously attempted is the immobilisation of an enzyme on polymeric substrates to prevent mobility and self-reactivity. Examples of immobilised enzyme are PEG-papain and chitosan-papain. Whereas the stability of proteolytic activity may be improved by such methods, the enzyme may be chemically altered in an irreversible way and accordingly may not exhibit sufficient activity on specific substrates, especially when applied to complex substrates such as skin and cellular proteins. Moreover, the altered chemical structure of such enzymes could result in deleterious reactions for the end user such as allergies and intolerances.

In order to address these problems, studies underpinning the present invention investigated the loss of proteolytic activity of proteases extracted from various sources, such as the *Carica papaya* (papaya) plant (including papain), and the *Ananas comosus* (pineapple) plant (including bromelain). Based on these studies, methods were developed for the enhancement and stabilisation of the proteolytic activity of proteases, particularly in pharmaceutical and cosmetic/cosmeceutical compositions containing proteases derived from the papaya and pineapple plants.

Surprisingly and unexpectedly, it was found that several of the methods that enhanced the stability of proteolytic activity also significantly enhanced proteolytic activity itself.

### Summary of the Invention

The present invention teaches new methods for the enhancement and stabilisation of the proteolytic activity of proteases. In particular embodiments, the protease is obtained from or is obtainable from a fruit and/or vegetable. Accordingly, in some embodiments, the protease is obtained from a fruit and/or vegetable, while in other embodiments, the protease is obtained from a recombinant expression system. In particular embodiments, the protease is a cysteine protease. In particular embodiments, the cysteine protease may be papain (EC 3.4.22.2), chymopapain (EC 3.4.22.6), bromelain (stem bromelain - EC 3.4.22.32 and fruit bromelain - EC 3.4.22.33), ficain (EC 3.4.22.3) or actinidain (EC 3.4.22.14). In particular embodiments, the protease is obtained from or obtainable from the *Carica papaya* (papaya) plant or the *Ananas comosus* (pineapple) plant. In particular embodiments, the protease is papain or bromelain.

In a first aspect, the present invention provides a method for the enhancement and/or stabilisation of the proteolytic activity of a protease, wherein the method comprises (i) contacting a protease with a reducing agent, wherein a cysteine residue of the protease is maintained in a reduced state and (ii) removing substantially all oxygen gas from the area surrounding the protease. In one embodiment, the oxygen gas is removed by degassing the preparation. In one embodiment, the protease is packaged in a substantially oxygen-free atmosphere.

In a related aspect the present invention provides a method for the enhancement and/or stabilisation of the proteolytic activity of a protease, wherein the method comprises (i) providing a solution or gel comprising the protease; (ii) contacting the protease in the solution or gel with a reducing agent, wherein a cysteine residue of the protease is maintained in a reduced state and (iii) removing substantially all oxygen gas from the solution or gel.

In a second aspect, the present invention provides a method for producing a composition comprising a protease with proteolytic activity that has been enhanced and/or stabilised, wherein the method comprises (i) contacting a protease with a reducing agent, wherein a cysteine residue of the protease is maintained in a reduced state and (ii) removing substantially all oxygen gas from the area surrounding the composition. In one embodiment, the oxygen gas is removed by degassing the composition. In one embodiment, the composition is packaged in a substantially oxygen-free atmosphere.

In a related aspect the present invention provides a method for producing a composition comprising a protease with proteolytic activity that has been enhanced and/or stabilised, wherein the method comprises (i) providing a solution or gel comprising the protease; (ii) contacting the protease in the solution or gel with a reducing agent, wherein a cysteine residue of the protease is maintained in a reduced state and (iii) removing substantially all oxygen gas from the solution or gel.

In a third aspect, the present invention provides a composition comprising one or more proteases with proteolytic activity that has been enhanced and/or stabilised, wherein the composition is obtained or obtainable by a method of the first aspect, such as a method comprising (i) contacting a protease with a reducing agent, wherein a cysteine residue of the protease is maintained in a reduced state and (ii) removing substantially all oxygen gas from the area surrounding the composition. In one embodiment, the oxygen gas is removed by degassing the composition. In one embodiment, the composition is packaged in a substantially oxygen-free atmosphere.

In a fourth aspect, the present invention provides a composition comprising one or more proteases and a reducing agent, wherein a cysteine residue of the protease is maintained in a reduced state, and wherein the composition is substantially oxygen free. In one embodiment, oxygen gas is removed by degassing the composition. In one embodiment, the composition is packaged in a substantially oxygen-free atmosphere.

According to the present invention, the enhancement and/or stabilisation of the proteolytic activity of proteases may also be achieved by immobilising a protease to an anionic polymeric matrix. The use of an anionic polymer enables a protease to be bound non-covalently to the matrix. This contrasts with a previous method in the art for immobilising proteases by forming covalent linkages with a matrix material, e.g. by reacting a portion of the primary amines of the protease with the carboxyl groups of a carbomer, where a portion of the remaining primary amines of the protease are crosslinked using an amine reactive crosslinking reagent.

Accordingly, in a fifth aspect, the present invention provides a method for the enhancement and/or stabilisation of the proteolytic activity of a protease, wherein the method comprises combining a protease and an anionic polymer matrix such that the protease is non-covalently bound to the anionic polymer matrix. In one embodiment, the polymer is a carbomer. In one embodiment, the composition is packaged in a substantially oxygen-free atmosphere.

In a sixth aspect, the present invention provides a method for producing a composition comprising a protease with proteolytic activity that has been enhanced and/or stabilised, wherein the method comprises combining a protease and an anionic polymer matrix such that the protease is non-covalently bound to the anionic polymer matrix. In one embodiment, the polymer is a carbomer. In one embodiment, the composition is packaged in a substantially oxygen-free atmosphere.

In a seventh aspect, the present invention provides a composition comprising one or more proteases with proteolytic activity that has been enhanced and/or stabilised, wherein the composition is obtained or obtainable by a method comprising combining a protease and an anionic polymer matrix such that the protease is non-covalently bound to the anionic polymer matrix. In one embodiment, the polymer is a carbomer. In one embodiment, the composition is packaged in a substantially oxygen-free atmosphere.

In an eighth aspect, the present invention provides a composition comprising one or more proteases and an anionic polymer matrix, wherein the protease is non-covalently bound to the anionic polymer matrix. In one embodiment, the polymer is a carbomer. In one embodiment, the composition is packaged in a substantially oxygen-free atmosphere.

The methods taught in the present invention can also be combined. For example, the method of the first aspect can be combined with the method of the fifth aspect, or the method of the second aspect can be combined with the method of the sixth aspect.

Accordingly, in a ninth aspect, the present invention provides a method for the enhancement and/or stabilisation of the proteolytic activity of a protease, wherein the method comprises (i) contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix. In one embodiment, the oxygen gas is removed by degassing the preparation. In one embodiment, the polymer is a carbomer. In one embodiment, the protease is packaged in a substantially oxygen-free atmosphere.

In one embodiment step (iii) is performed prior to steps (i) and (ii). In another embodiment, steps (i) to (iii) are performed in that order.

In a related aspect, the present invention provides a method for the enhancement and/or stabilisation of the proteolytic activity of a protease, wherein the method comprises (i) providing a solution comprising the protease; (ii) contacting the protease in the solution with a reducing agent, wherein a cysteine residue of the protease is maintained in a reduced state (iii) removing substantially all oxygen gas from the solution; and (iv) combining the protease and an anionic polymer matrix such that the protease is non-covalently bound to the anionic polymer matrix.

In one embodiment step (iv) is performed prior to steps (i) to (iii). In another embodiment, steps (i) to (iv) are performed in that order.

In a tenth aspect, the present invention provides a method for producing a composition comprising a protease with proteolytic activity that has been enhanced and/or stabilised, wherein the method comprises (i) contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix. In one embodiment, the oxygen gas is removed by degassing the preparation. In one embodiment, the polymer is a carbomer. In one embodiment, the composition is packaged in a substantially oxygen-free atmosphere.

In a related aspect, the present invention provides a method for producing a composition comprising a protease with proteolytic activity that has been enhanced and/or stabilised, wherein the method comprises (i) providing a solution or gel comprising the protease; (ii) contacting the protease in the solution or gel with a reducing agent, wherein a cysteine residue of the protease is maintained in a reduced state (iii) removing substantially all oxygen gas from the solution or gel; and (iv) combining the protease and an anionic polymer matrix such that the protease is non-covalently bound to the anionic polymer matrix.

In an eleventh aspect, the present invention provides a composition comprising one or more proteases with proteolytic activity that has been enhanced and/or stabilised, wherein the composition is obtained or obtainable by a method of the ninth aspect, such as a method comprising (i) contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix. In one embodiment, the oxygen gas is removed by degassing the preparation. In one embodiment, the polymer is a carbomer. In one embodiment, the composition is packaged in a substantially oxygen-free atmosphere.

In a twelfth aspect, the present invention provides a composition comprising one or more proteases, a reducing agent and an anionic polymer matrix, wherein a cysteine residue of the protease is maintained in a reduced state, the composition is substantially oxygen free and the protease is non-covalently bound to the anionic polymer matrix. In one embodiment, oxygen gas is removed by degassing the composition. In one embodiment, the polymer is a carbomer. In one embodiment, the composition is packaged in a substantially oxygen-free atmosphere.

In one embodiment, the composition is in the form of a gel.

In a thirteenth aspect, the present invention provides use of the compositions of the third, fourth, seventh, eighth, eleventh and/or twelfth aspects in the manufacture of a medicament. In some embodiments, the medicament is for the treatment of diseases and disorders including wounds. In some embodiments, the medicament is for debridement. In some embodiments, the medicament is for treating burns. In some embodiments, the medicament is for treating ulcers. In some embodiments, the medicament is for treating gangrene.

In a fourteenth aspect, the present invention provides use of the compositions of the third, fourth, seventh, eighth, eleventh and/or twelfth aspects in the manufacture of a cosmetic. In some embodiments, the cosmetic is for exfoliation, lightening skin, or for applying to wrinkles, skin blemishes, freckles, pimples, acne, rosacea, sun spots, scars or varicose veins, or for applying to dry, aged or damaged skin.

In a fifteenth aspect, the present invention provides a pharmaceutical composition comprising the compositions of the third, fourth, seventh, eighth, eleventh and/or twelfth aspects, together with a pharmaceutically acceptable carrier, diluent, excipient, surfactant and/or adjuvant.

In a sixteenth aspect, the present invention provides a cosmetic composition comprising the compositions of the third, fourth, seventh, eighth, eleventh and/or twelfth aspects, together with a cosmetically acceptable carrier, diluent, excipient, surfactant and/or adjuvant.

In a seventeenth aspect, the present invention provides the compositions of the third, fourth, seventh, eighth, eleventh and/or twelfth aspects, or the pharmaceutical composition of the fifteenth aspect, for use in the treatment of diseases and disorders including wounds. In some embodiments, the treatment is debridement. In some embodiments, the treatment is for burns. In some embodiments, the treatment is for ulcers. In some embodiments, the treatment is for gangrene. In some embodiments, the composition is applied topically.

In an eighteenth aspect, the present invention provides a method for the treatment of diseases and disorders including wounds, wherein the method comprises administering to a subject the compositions of the third, fourth, seventh, eighth, eleventh and/or twelfth aspects, or the pharmaceutical composition of the fifteenth aspect. In some embodiments, the treatment is debridement. In some embodiments, the treatment is for burns. In some embodiments, the treatment is for ulcers. In some embodiments, the treatment is for gangrene. In some embodiments, the composition is applied topically.

In a nineteenth aspect, the present invention provides the compositions of the third, fourth, seventh, eighth, eleventh and/or twelfth aspects, or the cosmetic composition of the sixteenth aspect, for use in lightening skin, exfoliation or for applying to wrinkles, skin blemishes, freckles, pimples, acne, rosacea, sun spots, scars or varicose veins, or for applying to dry, aged or damaged skin.

In a twentieth aspect, the present invention provides kits comprising the compositions of the third, fourth, seventh, eighth, eleventh and/or twelfth aspects, the pharmaceutical composition of the fifteenth aspect or the cosmetic composition of the sixteenth aspect. In one embodiment, the kits are used for performing the method of the eighteenth aspect or for the uses of seventeenth and nineteenth aspects.

In contrast to previously available compositions, the compositions of the present invention do not need to be lyophilised in order to be sufficiently stable for pharmaceutical or cosmetic uses. Therefore such compositions can be formulated, for example, as capsules, tablets, creams, ointments, solutions, pastes, drops, sprays, aerosols, vapours, wipes, patches, gauzes, gels or liquids. Accordingly, in one embodiment, the compositions of the present invention are provided or packaged as capsules, tablets, creams, ointments, solutions, pastes, drops, sprays, aerosols, vapours, wipes, patches, gauzes, gels or liquids and do not need to be reconstituted prior to use. In a preferred embodiment, the compositions of the present invention are provided or packaged in gel or liquid form and do not need to be reconstituted prior to use.

### Brief Description of the Figures

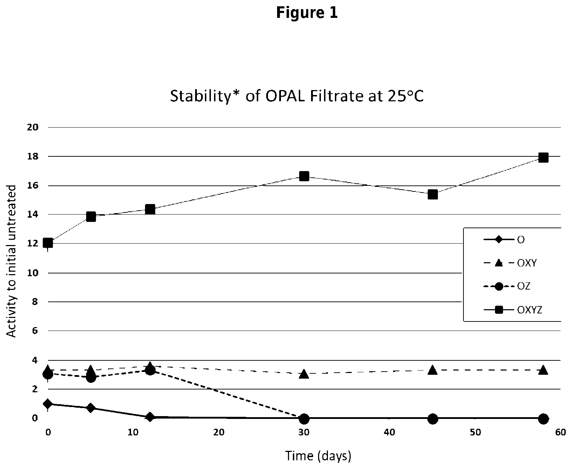

Figure 1. Stability of Opal with X+Y, Z and X+Y+Z treatments. Shows total proteolytic activity as measured by BApNA assay following X+Y, Z and X+Y+Z treatments. Ratios to initial Opal activity (2E3 USP units/mL). Activities at t=0 reflect immediate enhancement of proteolytic activity by X+Y, Z and X+Y+Z treatments. All treatments were performed on the same batch of Opal.

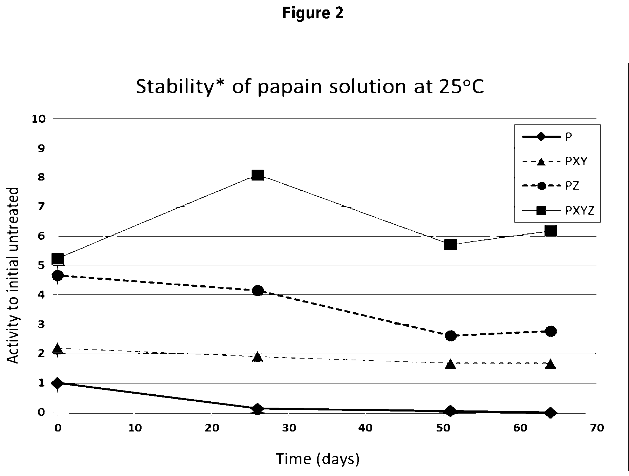

Figure 2. Stability of papain with X+Y, Z and X+Y+Z treatments. Shows total proteolytic activity as measured by BApNA assay following X+Y, Z and X+Y+Z treatments. Ratios to initial P activity (1E4 USP units/mL). Activities at t=0 reflect immediate enhancement of proteolytic activity by X+Y, Z and X+Y+Z treatments. All treatments were performed on the same batch of papain.

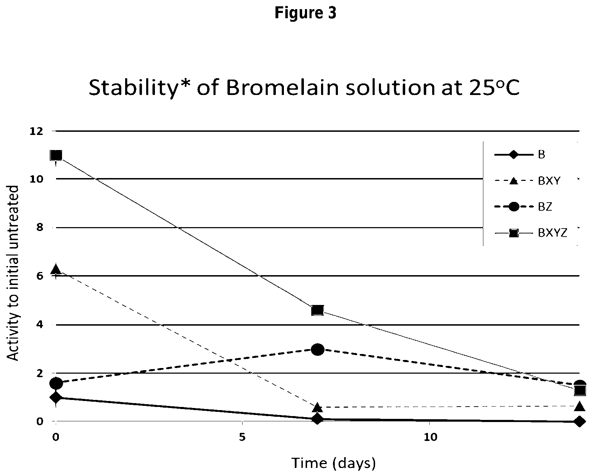

Figure 3. Stability of bromelain with X+Y, Z and X+Y+Z treatments. Shows total proteolytic activity as measured by BApNA assay following X+Y, Z and X+Y+Z treatments. Ratios to initial B activity (1.5E4 USP units/mL). Activities at t=0 reflect immediate enhancement of proteolytic activity by X+Y, Z and X+Y+Z treatments. All treatments were performed on the same batch of bromelain.

### Definitions

Unless defined otherwise, all technical and scientific terms used herein have the same meaning as commonly understood by those of ordinary skill in the art to which the invention belongs. Although any methods and materials similar or equivalent to those described herein may be used in the practice or testing of the present invention, preferred methods and materials are described. For the purposes of the present invention, the following terms are defined below.

The articles "a" and "*an*" are used herein to refer to one or to more than one (i.e. to at least one) of the grammatical object of the article. By way of example, "*an element*" means one element or more than one element.

By "*about*" is meant a quantity, level, value, number, frequency, percentage, dimension, size, amount, weight or length that varies by as much 20, 15, 10, 9, 8, 7, 6, 5, 4, 3, 2 or 1 % to a reference quantity, level, value, number, frequency, percentage, dimension, size, amount, weight or length.

Throughout this specification, unless the context requires otherwise, the words "*comprise*," "*comprises*" and "*comprising*" will be understood to mean the inclusion of a stated step or element or group of steps or elements but not the exclusion of any other step or element or group of steps or elements. Thus, use of the term "*comprising*" and the like indicates that the listed elements are required or mandatory, but that other elements are optional and may or may not be present. By "*consisting of*" is meant including, and limited to, whatever follows the phrase "*consisting of*". Thus, the phrase "*consisting of*" indicates that the listed elements are required or mandatory, and that no other elements may be present. By "*consisting essentially of*" is meant including any elements listed after the phrase, and limited to other elements that do not interfere with or contribute to the activity or action specified in the disclosure for the listed elements. Thus, the phrase "*consisting essentially of*" indicates that the listed elements are required or mandatory, but that other elements are optional and may or may not be present depending upon whether or not they affect the activity or action of the listed elements.

The term "*debridement*" refers to the removal of dead and damaged tissue from a wound.

The term "*obtainable*", when used in relation to compositions of the present invention, includes compositions produced not only by a particular specified method, but also the same compositions however produced, for example, by sourcing proteases from fruits or vegetables, or by recombinant DNA technology or other genetic engineering methods, for example, by using a recombinant expression system.

By "*isolated*" is meant material that is substantially or essentially free from components that normally accompany it in its native state. For example, an "*isolated protease*," as used herein, refers to *in vitro* isolation and/or purification of a peptide or polypeptide protease molecule from its natural cellular environment, and from association with other components of the cell, i.e., it is not associated with *in vivo* substances.

The term "*Opal*" refers to a papain-containing composition obtained from papaya fruit (not including the latex of the skin of the fruit). Opal may be prepared, for example, by the methods disclosed in international patent application no. PCT/AU2003/000931, published as WO2004/008887, the entire contents of which are incorporated herein by reference.

The terms "*patient*" "*subject*" and "*individual*" are used interchangeably and refer to patients, subjects and individuals of human or other mammals and includes any one for whom it is desired to a treat, prevent, ameliorate, or reduce the severity of a disease, disorder or condition using the invention. However, it will be understood that "*patient*" does not imply that symptoms are present. Suitable mammals that fall within the scope of the invention include, but are not restricted to, primates (e.g. humans, chimpanzees) livestock animals (e.g. sheep, cows, horses, donkeys, pigs), laboratory test animals (e.g. rabbits, mice, rats, guinea pigs, hamsters), companion animals (e.g. cats, dogs) and captive wild animals (e.g. foxes, deer, dingoes).

The phrase "*substantially oxygen-fr*ee", throughout this specification, includes a concentration of less than 1 ppm.

The phrases "*enhancement and*/*or stabilisation of the proteolytic activity of a protease*" and "*proteolytic activity that has been enhanced and*/*or stabilised*" and equivalent phrases as used herein refer to methods for the enhancement of the proteolytic activity of proteases and methods for stabilisation of the proteolytic activity of proteases, such as by increasing, preserving, prolonging or slowing the decline in the ability of a protease to perform its usual enzymatic function relative to a given unit of time, or relative to a reference level of activity. Such a method may therefore result in a protease composition that performs its enzymatic function in less time than it would take to perform its enzymatic function in the absence of the method. Alternatively, the method may result in the protease performing its enzymatic function at a higher level of activity than it would in the absence of the method, for example, when measured at particular time point or time points after the method has been undertaken. The less time taken or the higher level of activity may also be measured relative to a range of other factors, including, but not limited to, exposure to heat and sterilising radiation, as well as the storage time and storage conditions of the protease composition, and in addition any transportation conditions, including temperature, humidity and atmospheric pressure.

Enhancement and/or stabilisation of the proteolytic activity of a protease may be measured or confirmed, for example, by comparing the enzymatic activity of a protease subject to the methods of the present invention (for example, "sample A") with the stability of a protease that has not been subjected to the methods of the present invention (for example, a "control sample", which may for example be a wild type or naturally occurring protease). Such a comparison between samples can be undertaken, for example, at a specified time after the methods of the present invention have been undertaken in order to ascertain the increase in stability and/or activity of a "sample A" protease relative to a control sample.

The terms "*wild-type*" and "*naturally occurring*" are used interchangeably to refer to a gene product (e.g., a polypeptide such as a protease) that is most frequently observed in a population and is thus arbitrarily designated the "*normal*" or "*wild-type*" form of the gene.

The term "*wound*" means an injury to living tissue in which the skin is cut or broken and includes dermal ulcers and burns. Dermal ulcers may include diabetic ulcers, pressure ulcers, venous (or varicose) ulcers and arterial ulcers.

The reference to any prior art in this specification is not, and should not be taken as an acknowledgement or any form of suggestion that prior art forms part of the common general knowledge of the person skilled in the art.

The entire content of all publications, patents, patent applications and other material recited in this specification is incorporated herein by reference.

### Detailed Description of the Invention

Cysteine proteases, also known as thiol proteases and cysteine endopeptidases (EC 3.4.22), are enzymes that degrade proteins. They share a common catalytic mechanism that involves a nucleophilic cysteine thiol in a catalytic triad or dyad. They occur in a variety of organisms. In particular, they are commonly found in fruits including papaya (*Carica papaya and Vasconcellea cundianmarcensus*), pineapple (*Ananas comosus*), fig (*Ficus carica*) and kiwifruit (*Actinidia chinensis*) but may also be found in a variety of other fruits and vegetables. Cysteine proteases may be obtained by various methods including extraction from biological materials such as a fruit extract, e.g. from papaya pulp, or by recombinant expression in a suitable host cell. In one embodiment, the protease present in the compositions of the present invention or subject to the methods of the invention is a protease prepared from the ripe flesh of papaya, such as by the method described in WO2004/008887. In another embodiment the protease is bromelain (EC 3.4.22.33).

### Stabilisation Procedure

In one aspect of the present invention, a composition comprising a cysteine protease is subject to treatment with a reducing agent which maintains the active site cysteine amino acid residue of the protease in a reduced state. Cysteine residues when oxidised can form disulphide bridges which disrupt enzymatic activity. The reducing agent can help keep the active site residue in the reduced form, and also convert cysteine residues that have already become oxidised into the reduced form. Suitable reducing agents are known in the art and include cysteine. The amount of reducing agent added should generally be sufficient to regenerate all or most of the active site cysteine amino acid residues of the protease in solution and maintain them in the reduced form. This will typically involve adding the reducing agent in excess. In one embodiment, the concentration of the reducing agent, such as cysteine, is typically from 10 to 200 mM, such as from 50 to 150 mM. In particular embodiments, the concentration of the reducing agent, such as cysteine, is from 60 to 140mM, 70 to 130mM, 80 to 120mM, 90 to 100mM, 92 to 108mM, 94 to 106mM, 96 to 104 mM or 98 to 102mM. In one particular embodiment, the concentration of the reducing agent, such as cysteine, is about 100mM or 100mM.

The composition may for example be in the form of a liquid or gel comprising the protease. In one embodiment, the protease has previously been combined with an anionic polymer as described below prior to the above treatment step with a reducing agent.

The composition may also be subject to a step which removes substantially all of the oxygen present in the area surrounding the protease, for example substantially all of the oxygen **present** in a liquid or gel, such as a solution, which contains the protease. This can for example be achieved by degassing the composition, such as by flushing the composition with an inert gas such as nitrogen or argon. For example, nitrogen or argon purging for 20-40 min at flow rate of 25 mL/s can achieve residual dissolved oxygen of about 0.2-0.4 ppm. Other methods of removing substantially all of the oxygen present in the composition may also be employed, and will be known to the person skilled in the art, such as, but not limited to, heating at atmospheric pressure or under reduced pressure, or sonication at atmospheric pressure or under reduced pressure. The step of oxygen removal can be performed prior to, at the same time as, or immediately after the addition of the reducing agent.

The resulting composition comprises a cysteine protease and a reducing agent which reduces one or more active site cysteine residues of the cysteine protease, wherein the composition is substantially oxygen-free. At least some of the reducing agent may be in an oxidised state as a result of reactions with the cysteine protease and/or other components in the composition.

To reduce contact with oxygen during storage, the composition can be packaged to reduce or prevent oxygen absorption. For example, the composition may be packaged in a substantially oxygen free atmosphere, for example in a container with an inert gas such as nitrogen or argon. Alternatively, the composition may be vacuum packaged.

In another aspect, which can also be combined with the aforementioned aspects of the present invention, an anionic polymer matrix can be combined with a cysteine protease so that the cysteine protease is non-covalently bound to the anionic polymer matrix. This is intended to separate the enzyme molecules in a non-covalent way and thus limit autolysis and enzyme inactivation. The choice of anionic polymer is generally based on the high isoelectric point (pI) of papain and related proteases, which means that they are positively charged at neutral pH.

The anionic polymer may also be selected such that it will form a gel, for example at a pH between 6.5 and 8 or at alkaline pH.

In one embodiment, the anionic polymer matrix is a polyacrylic acid, such as a homopolymer, copolymer or interpolymer of acrylic acid. The anionic polymer typically has a high molecular weight. Examples of homopolymers are polymers of acrylic acid which are crosslinked with any of several polyalcohol allyl ethers (e.g., allyl ether pentaerythritol, allyl ether of sucrose, or allyl ether of propylene). Examples of copolymers are polymers of acrylic acid and C10-C30 alkyl acrylate crosslinked with, e.g. allyl pentaerythritol. Specific examples of suitable anionic polymers are Carbopol, Carbopol Ultrez, Carbomer 910, Carbomer 934, Carbomer 934p, Carbomer 940, and Carbomer 941 (obtainable from Lubrizol).

The anionic polymer can be combined with the protease to yield a liquid suspension. The concentration of anionic polymer may be from 0.01 to 3% w/w or more, such as from 0.1 to 2% w/w. If combined with other aspects of the present invention, then if not already performed, the reducing agent can be added at this stage and oxygen can be removed, such as by nitrogen flushing. Alkali can then be added to cause gelation of the liquid suspension to yield a viscous gel, for example by adjusting the pH to from 7.5 to 8.

The three different treatment steps can be performed in different orders. For example the combination with the anionic polymer may be performed first and then the addition of a reducing agent/removal of oxygen in either order. Alternatively the addition of a reducing agent/removal of oxygen can be performed in either order followed by the combination with the anionic polymer. It would also be possible to perform the combination with the anionic polymer in between the reducing agent and removal of oxygen steps. The various steps may be performed sequentially or at the same time (or with some degree of overlap).

The compositions of the present invention typically exhibit greater stability of proteolytic activity than a corresponding untreated protease control sample, i.e. one that is not non-covalently bound to an anionic polymer and is stored under normal oxidising conditions e.g. at an atmospheric oxygen level and with no addition of a reducing agent. For example, some protease compositions of the present invention, particularly papain (P) and papaya extract (such as Opal), may exhibit at least 10, 15, 20, 25, 30, 35, 40, 45, 50, 55, 60, 65, 70, 75, 80, 85, 90 or 95% proteolytic activity after storage at room temperature and pressure for 1, 2, 3, 4, 5, 6, 7, 8, 9, 10, 11, 12, 13, 14, 15, 16, 17, 18, 19, 20, 25, 30, 35, 40, 45, 50, 55 or 60 days or more while others, particularly bromelain (B), may exhibit at least 10, 15, 20, 25, 30, 35, 40, 45, 50, 55, 60, 65, 70, 75, 80, 85, 90 or 95% proteolytic activity after storage at room temperature and pressure for 1, 2, 3, 4, 5, 6, 7, 8, 9, 10, 11, 12, 13 or 14 days or more. Measured activity may be compared to activity immediately following treatment according to a process of the invention.

In some embodiments, the initial proteolytic activity of the protease compositions of the present invention is greater than the untreated protease compositions, such as having at least 1.5-fold activity as compared with the untreated protease, for example at least 2, 2.5, 3, 3.5, 4, 4.5, 5, 5.5, 6, 6.5, 7, 7.5, 8, 8.5, 9, 9.5 or 10-fold activity. For comparison purposes, the initial activity can be measured immediately after the treatment steps have been performed.

Activity can be measured using a BApNA spectrophotometric assay: a BApNA solution (10 mM in DMSO) can be prepared alongside a reaction buffer (potassium phosphate 100 mM, potassium chloride 116 mM and EDTA 3 mM, pH 6). A premix can be made with 2 parts water, 2 parts reaction buffer and 1 part v/v BApNA. The spectrophotometer (e.g. Jasco V-630 UV-Vis spectrophotometer) can be blanked at 410 nm with the premix solution. 1/5 v/v water can then be added to both the control sample and to the positive sample and reading can be started immediately afterwards. Relative enzyme activity can be measured as the slope of the resulting line.

### Compositions

In contrast to previously available compositions, the compositions of the present invention do not need to be lyophilised in order to be sufficiently stable for pharmaceutical and/or cosmetic uses. Therefore such compositions can be formulated, for example, as capsules, tablets, creams, ointments, solutions, pastes, drops, sprays, aerosols, vapours, wipes, patches, gauzes, gels or liquids. Accordingly, in one embodiment, the compositions of the present invention are provided or packaged as capsules, tablets, creams, ointments, emulsions, solutions, pastes, drops, sprays, aerosols, vapours, wipes, patches, gauzes, gels or liquids and do not need to be reconstituted prior to use. In a preferred embodiment, the compositions of the present invention are provided or packaged in gel or liquid form and do not need to be reconstituted prior to use.

In particular aspects of the invention, the compositions may be in the form of a gel or liquid. The improved stability of the proteolytic activity of the composition enables it to be provided in liquid or gel form which means it is ready to use and does not need to be reconstituted immediately prior to use. This is highly beneficial in the administration of the composition and may be useful in situations where it would be difficult to reconstitute a lyophilised composition, e.g. outside of a clinical setting where wounds, such as burns, need immediate treatment.

The compositions may also be absorbed into/adsorbed onto a solid material, such as a wound dressing. The compositions may also be combined with pharmaceutically and/or cosmetically carriers, diluents, excipients, surfactants and/or adjuvants to obtain a pharmaceutical or cosmetic composition of the invention. The compositions of the present invention may therefore be formulated to include one or more additional ingredients.

For example, surfactants may be used as part of the protease composition in order to improve physical characteristics of the composition. The presence of a surfactant does not affect the efficacy of the composition. The composition may therefore also incorporate any suitable surfactant such as an anionic, cationic or non-ionic surfactant such as sorbitan esters or polyoxyethylene derivatives thereof. Suitable surfactants may also include sodium dodecyl sulphate (SDS), ammonium lauryl sulphate, sodium laureth sulphate, and sodium myreth sulphate. Surfactants are commonly used to decrease non-specific adsorption, and require careful selection and optimisation. Suspending agents such as natural gums, cellulose derivatives or inorganic materials such as silicaceous silicas, and other ingredients such as lanolin, may also be included.

Compositions may be prepared according to methods which are known to those of ordinary skill in the art and may also include additional carriers, excipients or diluents. Carriers, excipients and diluents must be "acceptable" in terms of being compatible with the other ingredients of the composition, and not deleterious to formation of a composition that will be able to be stored for longer periods of time if required. Such carriers, excipients and diluents may be used for further enhancing the integrity and half- life of the compositions of the present invention.

Further examples of acceptable carriers or diluents are demineralised or distilled water; saline solution; vegetable based oils such as peanut oil, safflower oil, olive oil, cottonseed oil, maize oil, sesame oils, arachis oil or coconut oil; silicone oils, including polysiloxanes, such as methyl polysiloxane, phenyl polysiloxane and methylphenyl polysolpoxane; volatile silicones; mineral oils such as liquid paraffin, soft paraffin or squalane; cellulose derivatives such as methyl cellulose, ethyl cellulose, carboxymethylcellulose, sodium carboxymethylcellulose or hydroxypropylmethyl-cellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3-butylene glycol or glycerin; fatty acid esters such as isopropyl palmitate, isopropyl myristate or ethyl oleate; polyvinylpyrolidone; agar; gum tragacanth or gum acacia, and petroleum jelly. Carriers such as polyvinylpyrrolidone (PVP), carboxymethyl cellulose, polyvinyl alcohol, and polyethylene oxide may also be used.

Additional carriers that may be included in the compositions of the present invention include non-reducing sugars such as sucrose and reducing sugars such as lactulose. Such carriers, as well as sugar alcohols such as mannitol, xylitol, glycerol and sorbitol, are also useful for inclusion in the compositions of the present invention as they can act as antioxidants and potential stabilisers.

Methods for preparing administrable compositions are apparent to those skilled in the art, and are described in more detail in, for example, Remington's Pharmaceutical Science, 15th ed., Mack Publishing Company, Easton, Pa., incorporated by reference herein.

### Uses

The compositions of the present invention can be used in a variety of pharmaceutical applications related to the treatment of diseases, disorders and conditions, including skin conditions and wounds. The compositions of the present invention can also be used in a variety of cosmetic applications.

Accordingly, the present invention further provides methods of debridement of wounds comprising the topical application of a composition of the invention, compositions of the present invention for use in a method of treating wounds, methods of treating an individual suffering from burns, wherein the method comprises administering topically, or by other routes of administration, to an affected area of the individual a preparation of the invention, compositions of the invention for use or when used in a method of treating wounds, methods of enhancing wound healing, comprising administering topically, or by other routes of administration, to a wound a composition of the invention, methods of exfoliating or lightening skin comprising applying to the skin a cosmetic composition of the invention, uses of a cosmetic composition of the invention to exfoliate or lighten skin, methods of treating dry, aged or damaged skin comprising applying to the skin a cosmetic composition of the invention, and uses of a cosmetic composition of the invention to treat dry, aged or damaged skin.

The protease compositions of the present invention may in particular be used to prevent, treat, reduce or ameliorate a variety of skin conditions including wounds, including chronic wounds such as vascular/pressure skin ulcers, burns, and other skin conditions including but not limited to, eczema, psoriasis, acne, rosacea, ichthyosis, vitiligo, hives, seborrheic dermatitis.

Compositions of the present invention may be administered therapeutically or cosmetically. In such applications, compositions may be administered to a subject already suffering from a condition, in an amount sufficient to cure or at least partially arrest the condition and any complications. The quantity of the composition should be sufficient to effectively treat the patient.

The compositions may also be administered in the form of liposomes. Liposomes may be derived from phospholipids or other lipid substances, and may be formed by mono- or multilamellar hydrated liquid crystals dispersed in an aqueous medium. Any non-toxic, physiologically acceptable and metabolisable lipid capable of forming liposomes may be used. The compositions in liposome form may contain stabilisers, preservatives and excipients. Preferred lipids include phospholipids and phosphatidyl cholines (lecithins), both natural and synthetic. Methods for producing liposomes are known in the art, and in this regard specific reference is made to: Prescott, Ed., Methods in Cell Biology, Volume XIV, Academic Press, New York, N.Y. (1976), p. 33 et seq., the contents of which are incorporated herein by reference.

### Dosages

The "therapeutically effective" dose level for any particular patient will depend upon a variety of factors including the condition being treated and the severity of the condition, the activity of the composition employed, the age, body weight, general health, sex and diet of the patient, the time of administration, the route of administration, the duration of the treatment, and any drugs used in combination or coincidental with the treatment, together with other related factors well known in the art. One skilled in the art would therefore be able, by routine experimentation, to determine an effective, non-toxic amount of the composition which would be required to treat applicable conditions.

Further, it will be apparent to one of ordinary skill in the art that the optimal quantity and spacing of individual dosages of the composition will be determined by the nature and extent of the condition being treated, the form, route and site of administration, and the nature of the particular individual being treated. Also, such optimum conditions can be determined by conventional techniques.

It will also be apparent to one of ordinary skill in the art that the optimal course of treatment, such as the number of doses of the composition given per day for a defined number of days, can be ascertained by those skilled in the art using conventional course of treatment determination tests.

### Routes of administration

The compositions of the present invention can be administered by standard routes. In general, the compositions may be administered by topical routes. Typically, compositions of the present invention are administered topically to the affected area of an individual.

In other embodiments, the compositions may be administered by other enteral/enteric routes, such as rectal, sublingual or sublabial, or via the central nervous system, such as through epidural, intracerebral or intracerebroventricular routes. Other locations for administration may include via epicutaneous, transdermal, intradermal, nasal, intraarterial, intracardiac, intraosseus, intrathecal, intraperitoneal, intravesical, intravitreal, intracavernous, intravaginal or intrauterine routes.

### Timing of Therapies

Typically, in therapeutic applications, the treatment would be for the duration of the disease state.

Those skilled in the art will appreciate that the compositions disclosed herein may be administered as a single agent or as part of a combination therapy approach to the methods disclosed herein, either at diagnosis or subsequently thereafter, for example, as follow-up treatment or consolidation therapy as a compliment to currently available therapies for such treatments. The compositions disclosed herein may also be used as preventative therapies for subjects who are genetically or environmentally predisposed to developing such diseases.

The compositions may be administered regularly for as long as is needed, such as until an improvement in the condition is seen. Therefore they may be administered hourly, multiple times a day, daily, multiple times a week, weekly, monthly or at whatever frequency is deemed appropriate.

### Kits

Kits of the present invention facilitate the employment of the methods and uses of the present invention. Typically, kits for carrying out a method or use of the invention contain all the necessary reagents and means to carry out the method. For example, in one embodiment, the kit may comprise a composition of the present invention and, optionally, means to administer the composition such as devices for point of care methods.

Typically, the kits described herein will also comprise one or more containers. In the context of the present invention, a compartmentalised kit includes any kit in which compositions are contained in separate containers, and may include small glass containers, plastic containers or strips of plastic or paper. Such containers may allow the efficient transfer of compositions from one compartment to another compartment whilst avoiding cross-contamination of compositions, and the addition of agents or solutions of each container from one compartment to another in a quantitative fashion.

Typically, a kit of the present invention will also include instructions for using the kit to perform the appropriate methods and uses.

Methods, uses, compositions and kits of the present invention are equally applicable to any animal, including humans, for example including non-human primate, equine, bovine, ovine, caprine, leporine, avian, feline and canine species. Accordingly, for application to different species, a single kit of the invention may be applicable, or alternatively different kits, for example containing compositions specific for each individual species, may be required.

The person skilled in the art will understand and appreciate that different features disclosed herein may be combined to form combinations of features that are within the scope of the present invention.

### Enumerated embodiments

1. A method for the enhancement and/or stabilisation of the proteolytic activity of a protease, wherein the method comprises (i) contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix.
2. The method of embodiment 2, wherein the protease is packaged in a substantially oxygen-free atmosphere.
3. A method for producing a composition comprising a protease with proteolytic activity that has been enhanced and/or stabilised, wherein the method comprises contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix.
4. The method of embodiment 3, wherein the composition is packaged in a substantially oxygen-free atmosphere.
5. The method of any one of embodiments 1 to 4, wherein oxygen gas is removed by degassing the preparation.
6. The method of any one of embodiments 1 to 5, wherein the polymer is a carbomer.
7. A composition comprising one or more proteases with proteolytic activity that has been enhanced and/or stabilised, wherein the composition is obtained or obtainable by a method comprising (i) contacting a protease with a reducing agent, wherein a cysteine residue of the protease is maintained in a reduced state, (ii) removing substantially all oxygen gas from area surrounding the protease and (iii) combining the protease and an anionic polymer matrix such that the protease is non-covalently bound to the anionic polymer matrix.
8. A composition comprising one or more proteases, a reducing agent and an anionic polymer matrix, wherein a cysteine residue of the protease is maintained in a reduced state, the composition is substantially oxygen free and the protease is non-covalently bound to the anionic polymer matrix.
9. The composition of either one of embodiments 7 or 8, wherein oxygen gas is removed by degassing the composition.
10. The composition of any one of embodiments 7 to 9, wherein the polymer is a carbomer.
11. The composition of any one of embodiments 7 to 10, wherein the composition is packaged in a substantially oxygen-free atmosphere.
12. A method for the enhancement and/or stabilisation of the proteolytic activity of a protease, wherein the method comprises (i) contacting a protease with a reducing agent, wherein a cysteine residue of the protease is maintained in a reduced state and (ii) removing substantially all oxygen gas from area surrounding the protease.
13. The method of embodiment 12, wherein the protease is packaged in a substantially oxygen-free atmosphere.
14. A method for producing a composition comprising a protease with proteolytic activity that has been enhanced and/or stabilised, wherein the method comprises (i) contacting a protease with a reducing agent, wherein a cysteine residue of the protease is maintained in a reduced state and (ii) removing substantially all oxygen gas from area surrounding the composition.
15. The method of any one of embodiments 12 to 14, wherein the oxygen gas is removed by degassing the composition.
16. The method of either one of embodiments 14 or 15, wherein the composition is packaged in a substantially oxygen-free atmosphere.
17. A composition comprising one or more proteases with proteolytic activity that has been enhanced and/or stabilised, wherein the composition is obtained or obtainable by a method comprising (i) contacting a protease with a reducing agent, wherein a cysteine residue of the protease is maintained in a reduced state and (ii) removing substantially all oxygen gas from area surrounding the composition.
18. A composition comprising one or more proteases and a reducing agent, wherein a cysteine residue of the protease is maintained in a reduced state, and wherein the composition is substantially oxygen free.
19. The composition of either one of embodiments 17 or 18, wherein oxygen gas is removed by degassing the composition.
20. The composition of any one of embodiments 17 to 19, wherein the composition is packaged in a substantially oxygen-free atmosphere.
21. A method for the enhancement and/or stabilisation of the proteolytic activity of a protease, wherein the method comprises combining a protease and an anionic polymer matrix such that the protease is non-covalently bound to the anionic polymer matrix.
22. A method for producing a composition comprising a protease with proteolytic activity that has been enhanced and/or stabilised, wherein the method comprises combining a protease and an anionic polymer matrix such that the protease is non- covalently bound to the anionic polymer matrix.
23. The method of either one of embodiments 21 or 22, wherein the polymer is a carbomer.
24. The method of any one of embodiments 21 to 23, wherein the composition is packaged in a substantially oxygen-free atmosphere.
25. A composition comprising one or more proteases with proteolytic activity that has been enhanced and/or stabilised, wherein the composition is obtained or obtainable by a method comprising combining a protease and an anionic polymer matrix such that the protease is non-covalently bound to the anionic polymer matrix.
26. A composition comprising one or more proteases and an anionic polymer matrix, wherein the protease is non-covalently bound to the anionic polymer matrix.
27. The composition of either one of embodiments 25 or 26, wherein the polymer is a carbomer.
28. The composition of any one of embodiments 25 to 27, wherein the composition is packaged in a substantially oxygen-free atmosphere.
29. Use of the composition of any one of embodiments 7 to 11, 17 to 20 or 25 to 28 in the manufacture of a medicament for the treatment of diseases and disorders including wounds, for debridement, or for treating burns, ulcers or gangrene.
30. Use of the composition of any one of embodiments 7 to 11, 17 to 20 or 25 to 28 in the manufacture of a cosmetic for lightening skin, exfoliation or for applying to wrinkles, skin blemishes, freckles, pimples, acne, rosacea, sun spots, scars or varicose veins, or for applying to dry, aged or damaged skin.
31. A pharmaceutical composition comprising the composition of any one of embodiments 7 to 11, 17 to 20 or 25 to 28 together with a pharmaceutically acceptable carrier, diluent, excipient, surfactant and/or adjuvant.
32. A cosmetic composition comprising the composition of any one of embodiments 7 to 11, 17 to 20 or 25 to 28 together with a cosmetically acceptable carrier, diluent, excipient, surfactant and/or adjuvant.
33. A composition of any one of embodiments 7 to 11, 17 to 20 or 25 to 28, or a pharmaceutical composition of embodiment 31, for use in the treatment of diseases and disorders including wounds, for debridement, or for treating burns, ulcers or gangrene.
34. The composition of embodiment 33, wherein the composition is applied topically.
35. A method for the treatment of diseases and disorders including wounds, for debridement, or for treating burns, ulcers or gangrene, wherein the method comprises administering to a subject the composition of any one of embodiments 7 to 11, 17 to 20 or 25 to 28, or the pharmaceutical composition of embodiment 31.
36. The method of embodiment 35, wherein the composition is applied topically.
37. A composition of any one of embodiments 7 to 11, 17 to 20 or 25 to 28, or a cosmetic composition of embodiment 32, for use in lightening skin, exfoliation or for applying to wrinkles, skin blemishes, freckles, pimples, acne, rosacea, sun spots, scars or varicose veins, or for applying to dry, aged or damaged skin.
38. A kit comprising the composition of any one of embodiments 7 to 11, 17 to 20 or 25 to 28, the pharmaceutical composition of embodiment 31 or the cosmetic composition of embodiment 32.
39. The kit of embodiment 38, for use or when used for the treatment of diseases and disorders including wounds, for debridement, or for treating burns, ulcers or gangrene, or for use in lightening skin, exfoliation or for applying to wrinkles, skin blemishes, freckles, pimples, acne, rosacea, sun spots, scars or varicose veins, or for applying to dry, aged or damaged skin.

The present invention will now be further described with reference to the following examples, which are illustrative only and non-limiting.

### EXAMPLES

Cysteine proteases are a family of proteases characterised by the presence of the amino acid cysteine in the active site. This sulphur containing amino acid is responsible, together with an accessory histidine residue, for proteolytic activity by attacking the amide carbonyl of the peptide bond to form a thioester which is later attacked by water to regenerate the cysteine and release a carboxylic acid.

Cysteine proteases are found in abundance in the latex of numerous plant species such as *Carica papaya* (papain, chymopapain, caricain, glycyl endopeptidase), the genus *Ficus* (ficins), and several members of the *Bromeliacea* family such as *Ananas comosus* (bromelain).

However, cysteine proteases suffer from a major weakness that limits their usefulness: cysteine is particularly sensitive to oxidation because of the relatively strong reducing potential of the thiol group (E⁰= -0.34 V). In the presence of oxygen or oxidising agents the thiol will therefore undergo a series of oxidation steps ultimately yielding a sulfonic acid group (-SO3-). Since none of these oxidised species preserves the catalytic activity towards the peptide bond, oxidation can inactivate a cysteine protease.

Another reason for poor proteolytic stability of proteases is autolysis. Because proteases are themselves proteins, they can be degraded by an active protease, thereby causing loss of proteolytic activity. This is true for all proteases including cysteine proteases, and does not require external agents such as oxygen. While not wishing to be bound by theory, the present invention may therefore prevent or inhibit autolysis.

The present studies have identified and tested a combination of methods that are shown to enhance and stabilise of the proteolytic activity of proteases. These methods therefore provide a solution to the problem of protease inactivation due to oxidative damage and autolysis.

The methods of the present invention involve, in one aspect, the addition of cysteine as a reducing agent to maintain an active site cysteine residue of a protease in a reduced state and substantial removal of oxygen gas from the area surrounding the protease, for example, by flushing the solution with a stable halogen gas such as nitrogen or argon.

In an additional or alternative aspect, the addition of an anionic polymer (such as crosslinked polyacrylate) was also tested as a means to separate proteases in a non- covalent way and thus limit autolysis. The choice of anionic polymer based on the high pl of papain and related proteases means that they are positively charged at neutral pH. Accordingly, the present invention may result in proteases being bound to a polymer by reversible, electrostatic interactions.

### MATERIALS AND METHODS

Papain and bromelain (technical grade) from plant latex were obtained from Sigma Aldrich. A papain-containing composition was obtained from papaya fruit (not including the latex of the skin of the fruit). Such papain-containing compositions such as Opal may be prepared, for example, by the methods disclosed in international patent application no. PCT/AU2003/000931, published as WO2004/008887, the entire contents of which are incorporated herein by reference.

Nitrogen gas was sourced from BOC Gases (UK) and Carbopol Ultrez from Lubrizol Inc (OH, USA). All other chemicals were purchased from Sigma Aldrich. Spectrophotometric readings were taken on a Jasco V-630 UV-Vis spectrophotometer.

Cysteine addition: cysteine was added to each sample to a final concentration of 100 mM.

Nitrogen flushing: samples were placed in a plastic vial and nitrogen was bubbled into the solution for 5 minutes at room temperature. Each vial was immediately closed to prevent oxygen entry.

Carbopol addition: Carbopol Ultrez was added to each solution to a final concentration of 0.25% yielding a liquid suspension. For oxygen-free samples, nitrogen flushing was done at this stage. The subsequent addition of NaOH 10 M (1:1000, final 10 mM) caused immediate gelation of the solution to yield a viscous gel.

BApNA spectrophotometric assay: a BApNA solution (10 mM in DMSO) was prepared alongside a reaction buffer (potassium phosphate 100 mM, potassium chloride 116 mM and EDTA 3 mM, pH 6).

A premix was made with 2 parts water, 2 parts reaction buffer and 1 part v/v BApNA. The spectrophotometer was blanked at 410 nm with this solution. 1/5 v/v water was then added to both the control sample and to the positive sample and reading was started immediately afterwards. Relative enzyme activity was measured as the slope of the resulting line.

### Key

Opal (or O) - Papaya extract; P - Papain; B - Bromelain, each assayed with:
- No treatment (O, P or B),
- Treatment Z - Carbopol (0.25%),
- Treatment XY - addition of cysteine (X) and degassing with nitrogen (Y), or
- Treatment XYZ - addition of cysteine (X) and degassing with nitrogen (Y) and Carbopol (0.25%)

Opal stabilisation: Freshly made Opal (16 mL) was split in 2x 8 mL aliquots. Each aliquot was then further split in two resulting in 4x4 mL samples (Opal, Opal+XY, Opal+Z, Opal+XY+Z). For the X samples, cysteine was added (12 mg/mL, 100 mM). For the Z samples, Carbopol was added (0.25%). The XY samples were degassed under nitrogen for 5 minutes. To the Opal+XY+Z sample 1:1000 NaOH 10 M was then added to a final pH 7.5 under N₂ flush. To the Opal+Z sample1:1000 NaOH 10 M was then added to a final pH 7.5.

Papain stabilisation: Papain 1 mg/mL (16 mL) was split in 2x 8 mL aliquots. Each aliquot was then further split in two resulting in 4x4 mL samples (P, P+XY, P+Z, P+XY+Z). For the X samples, cysteine was added (12 mg/mL, 100 mM). For the Z samples, Carbopol was added (0.25%). The XY samples were degassed under nitrogen for 5 minutes. To the P+XY+Z sample 1:1000 NaOH 10 M was then added to a final pH 7.5 under N₂ flush. To the P+Z sample 1:1000 NaOH 10 M was then added to a final pH 7.5.

Bromelain stabilisation: Bromelain 1 mg/mL (16 mL) was split in 2x 8 mL aliquots. Each aliquot was then further split in 2 resulting in 4x4 mL samples (B, B+XY, B+Z, B+XY+Z).

For the X samples, cysteine was added (12 mg/mL, 100 mM). For the Z samples, Carbopol was added (0.25%) The XY samples were degassed under nitrogen for 5 minutes. To the B+XY+Z sample 1:1000 NaOH 10 M was then added to a final pH 7.5 under N₂ flush. To the B+Z sample 1:1000 NaOH 10 M was added to a final pH 7.5.

### RESULTS AND DISCUSSION

### 1. Stabilisation of the proteolytic activity of proteases

In summary, it was found that treatment with XY and XYZ resulted in stabilisation of the proteolytic activity of proteases for the Opal and papain samples, relative to untreated Opal and papain samples. Treatment with Z also resulted in stabilisation of the proteolytic activity of proteases for the papain sample. This can be seen in Figures 1 and 2. Treatment with XY and XYZ showed less stabilisation of the proteolytic activity of proteases for the bromelain samples, relative to untreated bromelain samples. This can be seen in Figure 3. These results are discussed in further detail as follows.

### Papaya extract (Opal)

Stability of the papaya extract stabilised solutions was assayed weekly for a period of 2 months via the BApNA assay. Activities measured were then normalized to that of the Opal (untreated) sample at the start of the experiment and set to 1. Results are presented in Figure 1.

The stabilising effect of the cysteine/nitrogen flushing combination resulted in an undetectable loss of activity over the period considered (up to at least Day 58). This could be explained by the absence of oxygen keeping the reducing agent cysteine in its reduced state, thereby allowing it to continue in turn to keep the active cysteines in the cysteine protease active sites in their reduced form and preventing oxidation. Similarly, the stabilising effect of the Carbopol, while lasting less time than treatment with the cysteine/nitrogen flushing combination, was still significant (up to at least Day 12).

An additional consideration was the enhancement in enzyme activity over time in the XY+Z sample (discussed below under part 2 of the results).

### 1.1 Papain (P)

Results for commercial latex papain solutions are shown in Figure 2. The results observed are similar to the Opal sample in that the XY combination and the Z (Carbopol) treatment both result in stabilisation of the enzyme solution at 64 days. The XYZ treatment for papain also showed stabilisation of the enzyme solution at 64 days.

### 1.2 Bromelain (B)

The observed results for bromelain also follow that of Opal and papain in regard to the increase in activity immediately after treatment for XY and XYZ treatments. The immediate effect of cysteine/nitrogen plus Carbopol is extremely pronounced, reaching amplifications of over 10-fold compared to untreated bromelain (Figure 3). Further, stabilisation was achieved with the Z treatment at 14 days.

While the stabilisation of proteolytic activity dropped off for bromelain over the time period tested, there nevertheless remained a net stabilisation in proteolytic activity of bromelain when treated with all of Carbopol (0.25%) alone (Z), both cysteine and degassing with nitrogen (XY), and with all of cysteine, degassing with nitrogen and Carbopol (0.25%) (XYZ), at both Day 7 and Day 14, when compared with the untreated sample.

### 2. Enhancement of the proteolytic activity of proteases

Surprisingly and unexpectedly, it was also found that the methods that enhanced the stability of proteolytic activity also significantly enhanced proteolytic activity itself. In summary, it was found that treatment with Z, XY and XYZ all resulted in enhancement of the proteolytic activity of proteases for each of the Opal, papain and bromelain samples, relative to untreated samples. This can be seen in Tables 1, 2 and 3 below, where "Mean" refers to mean relative enzyme activity, "SD" refers to standard deviation, "Z" refers to treatment with Carbopol (0.25%), "XY" refers to treatment with cysteine (X) and degassing with nitrogen (Y), and "XYZ" refers to treatment with cysteine (X), degassing with nitrogen (Y) and Carbopol (0.25%) (Z). The sample treatments shown in each table all used the same protease from the same batch.

These results suggest an immediate activation of the proenzyme (i.e. transformation of the proenzyme to the enzyme). The cysteine effect may be due to the reducing/activating effect of cysteine on the putative proenzyme and on the reversibly oxidized species. The unexpected Carbopol effect could be interpreted as a disruption of autolysis by the polymer of enzyme aggregates.

### 2.1 Papaya extract (Opal)

Table 1 shows that treatment with Carbopol (0.25%) alone (Z) resulted in an immediate (Day 0) 3-fold increase in proteolytic activity relative to the untreated sample. The increase in activity peaked at Day 12, followed by a drop in activity to base levels for the remainder of the test period.

Treatment with both cysteine and degassing with nitrogen (XY) resulted in an immediate (Day 0) 3.3-fold increase in proteolytic activity relative to the untreated sample. This increase in activity remained sustained at around this level throughout the test period, right up to Day 58. The maximum increase in activity was observed at Day 12 (3.59-fold increase) and the minimum increase in activity was observed at Day 30 (3-fold increase).

Treatment with all of cysteine, degassing with nitrogen and Carbopol (0.25%) (XYZ) resulted in an immediate (Day 0) 12-fold increase in proteolytic activity relative to the untreated sample. Significantly, this enhancement in proteolytic activity further increased in time, with a 13.8-fold increase observed at Day 5, a 14.3-fold increase observed at Day 12, a 16.6-fold increase observed at Day 30, a 15.3-fold increase observed at Day 45, and a 17.9-fold increase observed at Day 58. The active enzymes (i.e. protease) associated with the proenzyme and/or other inactive forms of the enzyme therefore appear to increase with time.

**Table 1 - Enhancement of the proteolytic activity of Opal (O)**

| | **Day 0** | **Day 5** | **Day 12** | **Day 30** | **Day 45** | **Day 58** |
|---|---|---|---|---|---|---|
| **Untreated (Mean)** | 1.000 | 0.718 | 0.103 | 0.000 | 0.000 | 0.000 |
| **Untreated (SD)** | 0.050 | 0.035 | 0.005 | 0.001 | 0.001 | 0.001 |
| **Z (Mean)** | 3.077 | 2.821 | 3.333 | 0.000 | 0.000 | 0.000 |
| **Z (SD)** | 0.153 | 0.060 | 0.060 | 0.001 | 0.001 | 0.001 |
| **XY (Mean)** | 3.333 | 3.333 | 3.590 | 3.077 | 3.333 | 3.333 |
| **XY (SD)** | 0.166 | 0.166 | 0.179 | 0.153 | 0.166 | 0.166 |
| **XYZ (Mean)** | 12.051 | 13.846 | 14.359 | 16.667 | 15.385 | 17.949 |
| **XYZ (SD)** | 0.602 | 0.692 | 0.717 | 0.833 | 0.769 | 0.040 |

The enhanced proteolytic activity of Opal observed with the various treatments appears to last for a significant period of time. The treatment with Carbopol (0.25%) alone (Z) showed enhanced proteolytic activity for at least 12 days, while the treatment with both cysteine and degassing with nitrogen (XY), and with all of cysteine, degassing with nitrogen and Carbopol (0.25%) (XYZ), showed enhanced proteolytic activity for at least 58 days, being the entirety of the test period.

### 2.2 Papain (P)

Table 2 shows that treatment with Carbopol (0.25%) alone (Z) resulted in an immediate (Day 0) 4.6-fold increase in proteolytic activity relative to the untreated sample. The increase was close to maintained up to at least Day 26 (4.1-fold increase), followed by a drop in activity to around a 2.6-2.7-fold increase for the remainder of the test period, up to at least Day 64.

Treatment with both cysteine and degassing with nitrogen (XY) resulted in an immediate (Day 0) 2.1-fold increase in proteolytic activity relative to the untreated sample. This increase in activity then gradually dropped throughout the test period, with the increase in activity at Day 26 being observed as a 1.905-fold increase and the increase in activity being at Days 51 and 64 being observed as a 1.667-fold increase.

Treatment with all of cysteine, degassing with nitrogen and Carbopol (0.25%) (XYZ) resulted in an immediate (Day 0) 5.2-fold increase in proteolytic activity relative to the untreated sample. Significantly, this enhancement in proteolytic activity showed a further net increase in time, with a 8.0-fold increase observed at Day 26, a 5.7-fold increase observed at Day 51, and a 6.1-fold increase observed at Day 64.

**Table 2 - Enhancement of the proteolytic activity of papain (P)**

| | **Day 0** | **Day 26** | **Day 51** | **Day 64** |
|---|---|---|---|---|
| **Untreated (Mean)** | 1.000 | 0.129 | 0.048 | 0.000 |
| **Untreated (SD)** | 0.048 | 0.014 | 0.014 | 0.002 |
| **Z (Mean)** | 4.667 | 4.143 | 2.619 | 2.762 |
| **Z (SD)** | 0.048 | 0.048 | 0.238 | 0.048 |
| **XY (Mean)** | 2.190 | 1.905 | 1.667 | 1.667 |
| **XY (SD)** | 0.190 | 0.048 | 0.048 | 0.048 |
| **XYZ (Mean)** | 5.238 | 8.095 | 5.714 | 6.190 |
| **XYZ (SD)** | 0.143 | 0.048 | 0.095 | 0.143 |

The enhanced proteolytic activity of papain observed with the various treatments again appears to last for a significant period of time. The treatment with Carbopol (0.25%) alone (Z), with both cysteine and degassing with nitrogen (XY), and with all of cysteine, degassing with nitrogen and Carbopol (0.25%) (XYZ), each showed enhanced proteolytic activity for at least 64 days, being the entirety of the test period.

### 2.3 Bromelain (B)

Table 3 shows that treatment with Carbopol (0.25%) alone (Z) resulted in an immediate (Day 0) 1.6-fold increase in proteolytic activity relative to the untreated sample. The increase was close to maintained up to at least Day 14 (1.5-fold increase), with an increase in activity to a 3.0-fold increase at Day 7.

Treatment with both cysteine and degassing with nitrogen (XY) resulted in an immediate (Day 0) 6.3-fold increase in proteolytic activity relative to the untreated sample. This increase in activity then dropped throughout the test period, with the increase in activity at Day 7 being observed as a 0.6-fold increase and the increase in activity at Day 14 being observed as a 0.65-fold increase.

Treatment with all of cysteine, degassing with nitrogen and Carbopol (0.25%) (XYZ) resulted in an immediate (Day 0) 11-fold increase in proteolytic activity relative to the untreated sample. This enhancement in proteolytic activity then dropped over time, with a 4.6-fold increase observed at Day 7, and a 1.3-fold increase observed at Day 14.

**Table 3 - Enhancement of the proteolytic activity of bromelain (B)**

| | **Day 0** | **Day 7** | **Day 14** |
|---|---|---|---|
| **Untreated (Mean)** | 1.000 | 0.110 | 0.000 |
| **Untreated (SD)** | 0.040 | 0.409 | 0.001 |
| **Z (Mean)** | 1.600 | 3.000 | 1.500 |
| **Z (SD)** | 0.025 | 0.033 | 0.067 |
| **XY (Mean)** | 6.300 | 0.600 | 0.650 |
| **XY (SD)** | 0.022 | 0.067 | 0.108 |
| **XYZ (Mean)** | 11.000 | 4.600 | 1.300 |
| **XYZ (SD)** | 0.036 | 0.109 | 0.077 |

The immediate enhancement of proteolytic activity of bromelain observed with the various treatments is consistent with the same trend observed for both Opal and papain. While the increase in activity dropped off more quickly for bromelain than for Opal and papain, there nevertheless remained a net increase in proteolytic activity of bromelain when treated with all of Carbopol (0.25%) alone (Z), both cysteine and degassing with nitrogen (XY), and with all of cysteine, degassing with nitrogen and Carbopol (0.25%) (XYZ), for the entirety of the test period, when compared with the untreated sample.

### CONCLUSIONS

The data presented herein teaches that enhancement and stabilisation of the proteolytic activity of proteases can be achieved using each of the methods disclosed herein, that is, treatment of proteases with Carbopol (0.25%) alone (Z), treatment of proteases with both cysteine and degassing with nitrogen (XY), and treatment of proteases with all of cysteine, degassing with nitrogen and Carbopol (0.25%) (XYZ).

Surprisingly and unexpectedly, it was also found that the methods that enhanced the stability of proteolytic activity also significantly enhanced proteolytic activity itself. In summary, it was found that treatment with Z, XY and XYZ all resulted in enhancement of the proteolytic activity of proteases. In particular, enhancement of the stabilisation of the proteolytic activity by the XYZ treatment is highly pronounced.

The data presented herein therefore teaches that both stabilisation of the proteolytic activity of proteases and enhancement of proteolytic activity itself can be achieved using the methods disclosed herein.

The treatments disclosed herein involve reagents, the safety record of which is excellent and which do not involve chemical covalent modification of the enzyme, thus preventing consumer safety related problems such as allergies and regulatory challenges.

While this invention has been disclosed with reference to specific aspects, it is apparent that other aspects and variations of this invention may be devised by others skilled in the art without departing from the true spirit and scope of the invention. Features and embodiments in different sections can be combined *mutatis mutandis.*

The following numbered paragraphs define particular embodiments of the present disclosure:
1. A method for the enhancement and/or stabilisation of the proteolytic activity of a protease, wherein the method comprises (i) contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix.
2. The method of para. 2, wherein the protease is packaged in a substantially oxygen-free atmosphere.
3. A method for producing a composition comprising a protease with proteolytic activity that has been enhanced and/or stabilised, wherein the method comprises contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix.
4. The method of para. 3, wherein the composition is packaged in a substantially oxygen-free atmosphere.
5. The method of any one of paras. 1 to 4, wherein oxygen gas is removed by degassing the preparation.
6. The method of any one of paras. 1 to 5, wherein the polymer is a carbomer.
7. A composition comprising one or more proteases with proteolytic activity that has been enhanced and/or stabilised, wherein the composition is obtained or obtainable by a method comprising (i) contacting a protease with a reducing agent, wherein a cysteine residue of the protease is maintained in a reduced state, (ii) removing substantially all oxygen gas from the area surrounding the protease and (iii) combining the protease and an anionic polymer matrix such that the protease is non-covalently bound to the anionic polymer matrix.
8. A composition comprising one or more proteases, a reducing agent and an anionic polymer matrix, wherein a cysteine residue of the protease is maintained in a reduced state, the composition is substantially oxygen free and the protease is non-covalently bound to the anionic polymer matrix.
9. The composition of either one of paras. 7 or 8, wherein oxygen gas is removed by degassing the composition.
10. The composition of any one of paras. 7 to 9, wherein the polymer is a carbomer.
11. The composition of any one of paras. 7 to 10, wherein the composition is packaged in a substantially oxygen-free atmosphere.
12. Use of the composition of any one of paras. 7 to 11 in the manufacture of a medicament for the treatment of diseases and disorders including wounds, for debridement, or for treating burns, ulcers or gangrene.
13. Use of the composition of any one of paras. 7 to 11 in the manufacture of a cosmetic for lightening skin, exfoliation or for applying to wrinkles, skin blemishes, freckles, pimples, acne, rosacea, sun spots, scars or varicose veins, or for applying to dry, aged or damaged skin.
14. A pharmaceutical composition comprising the composition of any one of paras. 7 to 11 together with a pharmaceutically acceptable carrier, diluent, excipient, surfactant and/or adjuvant.
15. A cosmetic composition comprising the composition of any one of paras. 7 to 11 together with a cosmetically acceptable carrier, diluent, excipient, surfactant and/or adjuvant.
16. A composition according to any one of paras. 7 to 11 or a pharmaceutical composition of para. 14, for use in the treatment of diseases and disorders including wounds, for debridement, or for treating burns, ulcers or gangrene.
17. The composition of para. 16, wherein the composition is applied topically.
18. A method for the treatment of diseases and disorders including wounds, for debridement, or for treating burns, ulcers or gangrene, wherein the method comprises administering to a subject the composition of any one of paras. 7 to 11 or the pharmaceutical composition of para. 14.
19. The method of para. 18, wherein the composition is applied topically.
20. A composition according to any one of paras. 7 to 11 or a cosmetic composition of para. 15, for use in lightening skin, exfoliation or for applying to wrinkles, skin blemishes, freckles, pimples, acne, rosacea, sun spots, scars or varicose veins, or for applying to dry, aged or damaged skin.

## Claims

1. A method for the enhancement and/or stabilisation of the proteolytic activity of one or more proteases, wherein the method comprises
(i) contacting the one or more proteases with a reducing agent, wherein an active site cysteine residue of the one or more proteases is maintained in a reduced state,
(ii) removing substantially all oxygen gas from the area surrounding the one or more proteases and
(iii) combining the one or more proteases and an anionic polymer matrix, such that the one or more proteases are non-covalently bound to the anionic polymer matrix,
thereby enhancing and/or stabilising the proteolytic activity of the one or more proteases.

2. The method of claim 1, wherein the one or more proteases is selected from
(i) a protease having a catalytic mechanism that involves a nucleophilic cysteine thiol in a catalytic triad or dyad, and
(ii) a cysteine protease.

3. The method of claims 1 or 2, wherein the reducing agent is cysteine.

4. The method of any one of claims 1-3, wherein oxygen gas is removed by degassing the preparation.

5. The method of any one of claims 1-4, wherein the anionic polymer matrix is a carbomer.

6. A composition comprising one or more proteases with proteolytic activity that has been enhanced and/or stabilised, wherein the composition is obtained or obtainable by a method comprising
(i) contacting the one or more proteases with a reducing agent, wherein an active site cysteine residue of the one or more proteases is maintained in a reduced state,
(ii) removing substantially all oxygen gas from the area surrounding the one or more proteases and
(iii) combining the one or more proteases and an anionic polymer matrix, such that the one or more proteases are non-covalently bound to the anionic polymer matrix.

7. The composition of claim 6, wherein the one or more proteases is selected from
(i) a protease having a catalytic mechanism that involves a nucleophilic cysteine thiol in a catalytic triad or dyad, and
(ii) a cysteine protease.

8. The composition of claims 6 or 7, wherein the reducing agent is cysteine.

9. The composition of any one of claims 6-8, wherein oxygen gas is removed by degassing the preparation.

10. The composition of any one of claims 6-9, wherein the anionic polymer matrix is a carbomer.

11. A composition for use as a medicament comprising the composition of any one of claims 6-10, optionally together with a pharmaceutically acceptable carrier, diluent, excipient, surfactant and/or adjuvant.

12. Use of a composition of any one of claims 6-10, together with a cosmetically acceptable carrier, diluent, excipient, surfactant and/or adjuvant in cosmetic applications.

13. A composition according to any one of claims 6-10, for use in the treatment of wounds, for use in debridement, or for use in treating burns, ulcers or gangrene.

14. The composition for use of claim 13, wherein the composition is applied topically.

15. The use of claim 12, wherein the cosmetic applications are selected from lightening skin, exfoliation or for applying to wrinkles, skin blemishes, freckles, pimples, acne, rosacea, sun spots, scars or varicose veins, or for applying to dry, aged or damaged skin.
